# EUROPÄISCHE PATENTSCHRIFT

(11) **EP 1 855 593 B1**
(45) Veröffentlichungstag und Bekanntmachung des Hinweises auf die Patenterteilung: **07.10.2009**
(21) Anmeldenummer: 06705873.5
(22) Anmeldetag: 01.02.2006
(51) Int. Cl.: A61B 5/22

(54) **VORRICHTUNG ZUR ERMITTLUNG VON KRAFT UND WEG SICH BEWEGENDER KÖRPER ODER KÄRPERTEILE IN HYGIENISCH GESCHLOSSENEM SYSTEM**
DEVICE FOR DETERMINING THE FORCE AND PATH OF A BODY OR BODY PARTS MOVING IN A HYGIENICALLY CLOSED SYSTEM
DISPOSITIF POUR DETERMINER LA FORCE ET LA TRAJECTOIRE DE CORPS OU DE PARTIES DE CORPS SE DEPLAÇANT DANS UN SYSTEME HYGIENIQUEMENT FERME

(30) Priorität: 11.02.2005 DE 102005006323
(43) Veröffentlichungstag der Anmeldung: 21.11.2007
(73) Patentinhaber: Arnold, Wolfgang, 45359 Essen (DE); Blattner, Farina, 45149 Essen (DE)
(72) Erfinder: Arnold, Wolfgang, 45359 Essen (DE); Blattner, Farina, 45149 Essen (DE)
(74) Vertreter: Schulte, Jörg
(86) Internationale Anmeldenummer: PCT/DE2006/000143
(87) Internationale Veröffentlichungsnummer: WO 2006/084430

(56) Entgegenhaltungen:
- DE-U1- 29 819 033
- US-B1- 6 190 335
- JAEGER K ET AL: "MEASURING MASTICATORY FORCES WITH STRAIN GAGES" REPORTS IN APPLIED MEASUREMENT, HOTTINGER BALDWIN MESSTECHNIK. DARMSTADT, DE, Bd. 7, Nr. 2, Januar 1991 (1991-01), Seiten 39-42, XP000249822 ISSN: 0930-7923

## Beschreibung

Die Erfindung betrifft eine Vorrichtung zur Ermittlung von Kraft und/oder Weg sich bewegender Gegenstände, Körper oder Körperteile bestehend aus einem Gehäuse mit eingelagerten, von einer Ausgleichsplatte beeinflussbaren Sensoren und einem auf die Ausgleichsplatte einwirkenden dem Gegenstand, Körper oder Körperteil zugeordneten Bewegungs- und/oder Kraftinitiator. Solch eine Vorrichtung ist bekannt aus DE 29819033 U.

Derartige Vorrichtungen werden eingesetzt, um die Bewegungsrichtung und den Bewegungsverlauf eines Gegenstandes oder Körpers oder Körperteils gegenüber einem festen Gehäuse zu überprüfen und zu ermitteln. Dies ist sowohl bei Sportlern wie auch auf anderen Gebieten interessant, um beispielsweise damit die Bewegungsabläufe zu überprüfen und ggf. zu korrigieren. Befindet sich beispielsweise der Bewegungs- und/oder Kraftinitiator über dem Mittelpunkt des Gehäuses, also praktisch zwischen den messenden Sensoren, so werden die Sensoren im gleichen Maße belastet, sodass ihre elektrischen Signale die gleiche Amplitude aufweisen und darüber entweder die Kraft oder die Position des Bewegungs- und/oder Kraftinitiators ermittelt werden kann. Verschiebt sich nun die Position oder die Spitze des Bewegungs- und/oder Kraftinitiators in Folge der gewollten oder ungewollten Bewegung, so werden die auf die Sensoren übertragenen Kräfte unterschiedlich beeinflusst und sie geben Signale mit unterschiedlicher Amplitude ab. Aus diesen Signalen lässt sich dann auf gezielte Weise die Lage des Bewegungs- und/oder Kraftinitiators auf der darunter befindlichen Ausgleichs-platte ermitteln. Auf diese Weise lässt sich sowohl die Kraft ermitteln, wie auch über die Zeit die zweidimensionale Bewegung gegenüber dem festen Punkt, also letztlich dem Gehäuse. Diese Ermittlungsart macht man sich auch zu Nutze bei der Ermittlung der Unterkieferbewegung und der Kieferschlusskraft bei entsprechend belasteten Patienten. Hierzu ist auf die DD-A-252 123 und DD 343 431 hinzuweisen. Nachteilig bei diesen bekannten Vorrichtungen allgemeiner aber auch auf dem medizinischen Gebiet ist, dass entweder eine ungenügende Möglichkeit der Aufzeichnung, Auswertung und Festschreibung der ermittelten Ergebnisse gegeben ist bzw. diese stark durch die verschiedensten Faktoren beeinträchtigt wird. Besonders nachteilig ist, dass die ermittelnden Sensoren nicht nur durch die bewegten Teile, also die sie aufnehmenden Träger beeinflusst werden können, sondern vor allem die Umgebung, die sowohl gasförmigen wie vor allen Dingen auch flüssigen Schadstoffen voll ausgesetzt sind. Vor allem beim Einsatz im medizinischen Bereich ist damit eine Gefahren- und Gefährdungsquelle vorgegeben, die aus hygienischen Gründen (Kontamination) nicht akzeptabel ist. Die ermittelten Ergebnisse können nicht nur negativ oder falsch beeinflusst werden, sondern die fehlende Hygiene stellt auch eine Gefährdung für den jeweiligen Patienten, insbesondere den zahnmedizinischen Patienten dar.

Der Erfindung liegt daher die Aufgabe zu Grunde, eine Vorrichtung zu schaffen, die auch in gas- und/oder flüssigkeitssäurebelasteten Räumen eingesetzt werden und die sowohl Bewegungen des Kraftinitiators wie dessen Druckkraft dauerhaft genau ermitteln kann.

Die Aufgabe wird erfindungsgemäß dadurch gelöst, dass die Sensoren einem mehrfach gekammerten Grundkörper aus elastischem Kunststoff zugeordnet sind, der durch eine spezielle Kurvengeometrie in Kombination mit einer bestimmten Shore-Härte die Funktion von Biegeelementen aufweisend ausgeführt ist, die die Sensoren aufnehmen und die zusammen mit den Sensoren von einem, das Gehäuse ergänzend ausfüllenden, elastischen Dichtkunststoffkörper eingefasst sind.

Mit einer derartigen Vorrichtung ist erstmals möglich, Kraft und/oder Weg sich bewegender Körper oder Körperteile sowie auch von Gegenständen genau zu ermitteln, auszuwerten, zu speichern und die benötigten Werte daraus in geeigneter Form zu nutzen. Dabei ist sichergestellt, dass sowohl die Sensoren wie die übrigen Teile des eigentlichen Messgerätes oder besser der Vorrichtung so verschlossen sind, dass sie zwar ihre Funktion voll erfüllen können, aber hygienisch einwandfrei einsetzbar bleiben. Sie können durch irgendwelche Umweltgase oder Umweltflüssigkeiten nicht beeinträchtigt werden, weil die gesamte Einheit so geschlossen und optimiert ausgebildet und untergebracht ist, dass eine bleibend sichere Funktion ohne Beeinflussung durch die Umwelt und ohne Beeinflussung der Umwelt erreicht ist. Die zum Einsatz kommenden elastischen Kunststoffe ermöglichen dabei einen einwandfreien Betrieb der Sensoren, die über Kabel oder drahtlos mit den zuständigen Messgeräten verbunden werden können. Der zum Einsatz kommende Grundkörper und der verschließende Dichtkunststoffkörper sind so geformt und aufeinander abgestimmt, dass die von der Ausgleichsplatte übertragenen Werte und Details sicher aufgenommen, gemessen und ausgewertet werden können. Auch dann, wenn eine derartige Vorrichtung in der Zahnmedizin im Speichel oder in sonstigen Körperhöhlen zum Einsatz kommt, also im Mund untergebracht wird, ist die Hygiene gesichert, weil der zum Einsatz kommende Kunststoff problemlos sterilisierbar und/oder desinfizierbar ist und dann auch lange Zeit steril bleibt, also eine hygienisch einwandfreie Funktion sichert und zwar auch dann, wenn die Mundflüssigkeit auf diese Vorrichtung einwirkt. Damit ist ein hygienisch einwandfreier Betrieb unabhängig vom jeweiligen Einsatzort gesichert und möglich, ohne dass bei längeren Standzeiten irgendwelche negativen Folgen zu befürchten sind.

Der zum Einsatz kommende, aus elastischem Kunststoff bestehende Grundkörper kann die über die Ausgleichsplatte übertragenen Kräfte und Bewegungen besonders gut aufnehmen und dabei die Sensoren entsprechend beeinflussen, wenn er mehrfach, zumindest dreifach gekammert ist. Dies bedeutet, dass bei einem ringförmigen Grundkörper zwar auch Messwerte ermittelt würden, aber in der Regel zu ungenaue, während bei der Dreifach-Kammerung oder Mehrfach-Kammerung bei den sich ändernden Belastungen bzw. Kräften auf den Grundkörper sich die Richtungen und Beeinflussungen leichter und deutlicher ermitteln lassen, als bei dem durchgehenden Grundkörper. Dabei werden die entsprechenden Sensoren den einzelnen Kammern oder Abschnitten des Grundkörpers zugeordnet und zwar so, dass die auftreffenden und über die Ausgleichsplatte übertragenen Kräfte auch gezielt auf die Sensoren übertragen werden.

Um den elastischen Kunststoff entsprechend sicher unterzubringen und andererseits die gewünschten und notwendigen Bewegungen ausführen und übertragen zu können, ist vorgesehen, dass das Gehäuse dosenförmig ausgebildet und eine feste Hülle darstellend aus Metall oder einem Hartkunststoff gefertigt ist. In einem solchen Gehäuse ist wie weiter vorne erwähnt sowohl der mehrfach gekammerte Grundkörper wie auch der umschließende aus dem gleichen oder ähnlichen Kunststoff hergestellte Dichtkunststoffkörper untergebracht ist und zwar so, dass die Sensoren dicht umschlossen und vor Umwelteinflüssen geschützt sind.

Als besonders zweckmäßig hat es sich herausgestellt, die Sensoren als Dehnungsmessstreifen auszubilden und auf den bogenförmigen Teil des Grundkörpers aufzubringen. Weiter vorne ist bereits erwähnt, dass der elastische Kunststoff des Grundkörpers eine spezielle Kurvengeometrie aufweist, sodass er in Verbindung mit der Shore-Härte eine genaue Übertragung der auf die Ausgleichsplatte und damit auf den "Messkörper" einwirkenden Kräfte sicher auf die Sensoren weitergibt. Vorteilhaft ist dabei, dass die Dehnungsmessstreifen sich auf diesen bogenförmigen Teil des Grundkörpers auflegen und dort festlegen lassen, wobei weiter hinten noch erläutert wird, wie das Festlegen letztlich erfolgen soll.

Das Fixieren und bleibende Festlegen der Dehnungsmessstreifen oder besser gesagt der Sensoren auf dem Grundkörper erreicht man insbesondere dadurch, dass die Dehnungsmessstreifen auf den bogenförmigen Teil des Grundkörpers aufgelegt, diesem angeformt und dann über das Kegelstück des Dichtkunststoffkörpers darauf fixiert sind. Je nach Ausbildung der einzelnen Kunststoffkörper kann schon durch das entsprechende Auflegen bzw. Aufpressen eine Verbindung erfolgen, sodass damit die Dehnungsmessstreifen auf Dauer wirksam festgelegt sind. Andererseits können die Werkstoffe, d. h. die Kunststoffe so aufeinander eingestellt werden, dass sie nacheinander aufgegossen oder auch aufgeklebt werden, um so gleichzeitig den wichtigen Dehnungsmessstreifen, d. h. die Sensoren auf Dauer vor der Umwelt geschützt festzulegen. Dabei bleiben die Anschlusspunkte für die Datenübertragungskabel außerhalb dieser Kunststoffumhüllung, sodass auch bei der nachfolgenden Montage zusätzliche Arbeiten dadurch nicht erforderlich sind. Da das Kegelstück und der eigentliche Grundkörper auch von der Form her aufeinander abgestimmt sind oder aufeinander abgestimmt geformt werden, bleiben die Dehnungsmessstreifen zwischen diesen beiden Körpern sicher fixiert.

Als vorteilhaft hat sich herausgestellt, wenn die Dehnungsmessstreifen mittig der Kammern auf den Grundkörper aufgebracht sind. Sie werden dann gleichförmig von den auf die Ausgleichsplatte einwirkenden Kräfte beeinflusst, sodass eine genaue Erfassung auch der Bewegungen gleichzeitig möglich ist.

Weiter vorn ist erwähnt worden, dass der Hohlraum oder der Innenraum des Gehäuses durch den elastischen Dichtkunststoffkörper ausgefüllt ist. Aus diesem Grunde ist vorzugsmäßig vorgesehen, dass der Dichtkunststoffkörper aus dem mittleren, in den Grundkörper eingepassten Kegelstück und dem den Grundkörper einfassenden Ringkörper besteht. Dieser Ringkörper sorgt für den dichten Anschluss an die Wände des Gehäuses, sodass eine genaue Anordnung der für die Messung wichtigen Teile innerhalb des Gehäuses dann sichergestellt ist. Der Ringkörper hat darüber hinaus noch die weitere Aufgabe, nämlich die Ausgleichsplatte mit zu fixieren, was dadurch erreicht wird, dass der Dichtkunststoffkörper oder dessen Ringkörper zugleich die Ausgleichsplatte fixierend eingebracht, vorzugsweise sie einklebend ausgebildet ist. Die Ausgleichsplatte schwimmt somit quasi in dem Dichtkunststoffkörper bzw. in dem entsprechenden elastischen Material, hat also die notwendige Bewegungsfreiheit und kann die Bewegung und die Kraft betreffenden Daten über das Kegelstück oder über den Grundkörper direkt auf die Dehnungsmessstreifen bzw. Sensoren übertragen.

Der von der Hygiene und der Wirkungsweise her optimale Grundkörper ist der, bei dem er und auch der Dichtkunststoffkörper aus kalthärtendem, additionsvernetzendem Silikon entsprechend abgestimmter Zusammensetzung hergestellt sind. Beide Kunststoffteile bestehen aus dem gleichen Grundmaterial, wobei bei Bedarf geringfügig unterschiedliche Eigenschaften in das jeweilige Silikon hinein gegeben werden und zwar durch entsprechende Additive, wobei aber gleichzeitig sichergestellt ist, dass beide Kunststoffkörper sich auch miteinander so verbinden und so aneinander kleben, dass sie den gewünschten "Gesamtkörper" innerhalb des Gehäuses ergeben. Das entsprechende Silikon wird aus Kartuschensystemen nahezu blasenfrei appliziert, sodass sich immer eine homogene und dichte Struktur für das Silikon ergibt, was die unbeeinflusste Weitergabe und Übertragung der Bewegungs- und Kraftdaten auf die jeweiligen Sensoren sicherstellt.

Die notwendige und gewünschte gezielte Aufnahme und Weitergabe der die Bewegung und Kraft betreffenden Informationen und Daten ist insbesondere dann gewährleistet, wenn der die Ausgleichsplatte fixierende Ringkörper ein dieser und dem Gesamtsystem die notwendige Biegefreiheit belassendes Elastizitätsmodul aufweist. Gleiches gilt nicht nur für den Ringkörper, sondern auch für das Kegelstück und letztlich auch für den Grundkörper. Bei entsprechender Biegefreiheit ist sichergestellt, dass die jeweiligen Verformungen die auftretenden Bewegungs- und Kraftdaten wiedergeben und so eine einwandfreie Übertragung und Auswertung der entsprechenden Daten durch die Sensoren möglich macht.

Die Ausgleichsplatte soll gemäß einer zweckmäßigen Weiterbildung der Erfindung als verwindungsfreie Membran aus Metall, Keramik und/oder Kunststoff bestehend ausgebildet sein, wobei schon wegen des Gewichtes in der Regel auch mit Aluminium oder ähnlichem Material gearbeitet werden kann. Wichtig ist, dass eine verwindungsfreie Ausbildung gewährleistet ist, was eben bei Metall, Keramik und Kunststoff entsprechender Ausführung gewährleistet ist.

Gerade in der Region oberhalb der Sensoren ist es wichtig, dass die Übertragung der auf die Ausgleichsplatte wirkenden Kräfte für die Ermittlung der Kraft und auch der Bewegung nicht durch irgendwelche Anhaftungen, Klebungen oder Ähnliches beeinträchtigt wird. Von daher ist es zweckmäßig, dass die Region oberhalb der vom Kegelstück eingefassten Sensoren eine hohe Gleitmöglichkeit aufweisend ausgebildet ist. Dabei ist zur Lösung vorgesehen, dass in den Regionen zwischen Ausgleichsplatte und Kegelstück eine Silikonöl-Schicht aufgebracht ist, die diese vorteilhafte Gleitwirkung garantiert. Denkbar ist auch, dass die gesamte Ausgleichsplatte quasi in einer Silikonöl-Schicht schwimmt, wobei dieses Schwimmen relativ zu sehen ist. Wichtig ist eben, dass eine direkte Verbindung zwischen Ausgleichsplatte und Kegelstück unterbunden ist, um sicherzustellen, dass die Kräfte auch wirksam weiter in das Kegelstück und damit in den Bereich der Sensoren geleitet werden.

Neben der Silikonöl-Schicht ist auch die Möglichkeit gegeben, oberhalb der Sensoren im Kegelstück kugelförmige oder kegelförmige Druckvermittler anzuordnen, über die sichergestellt wird, dass die Bewegungen auf der Ausgleichsplatte gezielt auf die unterhalb liegenden Sensoren weitergeleitet werden, wobei beispielsweise drei solcher Druckvermittler oberhalb der drei Sensoren bzw. Dehnungsmessstreifen positioniert sind, sodass nicht die gesamte Kammer für die Druckübermittlung benötigt wird, sondern diese Druckübermittlung punktuell erfolgen kann, dann allerdings wieder vergleichmäßigt durch das elastische Material des Grundkörpers.

Eine bleibend genaue Anordnung der Druckvermittler ist gewährleistet, wenn sie in Ausnehmungen im Kegelstück angeordnet sind, während die Ausgleichsplatte auf ihnen aufliegt. Hierdurch ist verhindert, dass eine zu ungleiche Verteilung der Kräfte erfolgt, die die Messergebnisse verschleiern könnten.

Die Rundumsicherung für die Sensoren innerhalb der Vorrichtung ist insbesondere dann gegeben, wenn der Ringkörper des Dichtkunststoffkörpers mit der Unterseite und dem Rand der Ausgleichsplatte und mit der innenwand des Gehäuses den Innenraum des Gehäuses voll abdichtend verklebt ist. Bei einer derartigen Ausbildung würde unter Umständen wie weiter oben schon erwähnt "Verneblung" der Messwerte eintreten, allerdings so vergleichmäßigt, dass daraus dennoch die nötigen genauen Ergebnisse zu ermitteln sind. Dafür wäre der Vorteil mit dieser Weiterbildung verbunden, den gesamten Innenraum wirksam abzudichten und so vor flüssigen und gasförmigen Schadstoffen wirksam zu schützen. Dabei muss beim Einsatz im zahnmedizinischen Bereich berücksichtigt werden, dass auch bei der Reinigung der entsprechenden Vorrichtung nach einem Einsatz mit chemischen Flüssigkeiten und Ähnlichem auf den Dichtkunststoffkörper und den Grundkörper eingewirkt wird, was dann ohne Einfluss auf den Messteil bleibt, wenn die hier geschilderte Abdichtung wirksam abgesichert ist.

Die Erfindung zeichnet sich insbesondere dadurch aus, dass eine Vorrichtung geschaffen ist, die auch in gas- und/oder flüssigkeitssäurebelasteten Räumen und Bereichen sicher eingesetzt werden kann, weil der messtechnische Teil von einem elastischen und hygienisch einwandfreien Kunststoff umgeben ist und so bleibend wirksam erhalten werden kann. Dabei werden sowohl Bewegungen des Kraftinitiators wie dessen Druckkraft dauernd genau ermittelbar, weil eben der messtechnische Teil innerhalb des Gehäuses so angeordnet und untergebracht ist, dass er durch die Umwelt völlig unbelastet bleibt. Diese besondere Anordnung und Ausbildung des Messteils der erfindungsgemäßen Vorrichtung hat dabei auch den Vorteil, dass die Messfunktion bleibend sicher erbracht werden kann, sodass lange Standzeiten derartiger Vorrichtungen zu erwarten sind. Die erfindungsgemäße Vorrichtung kann darüber hinaus in vielen Bereichen eingesetzt werden, um Bewegungen und die Kraft sich bewegender Körper und Körperteile sowie von Gegenständen zu ermitteln und festzuhalten, wobei sowohl an den Einsatz im Sportbereich wie auch im technischen sonstigen Bereich gedacht werden kann. Darüber hinaus eignet sich eine derartige Vorrichtung optimal auch zur intraoralen Ermittlung der zweidimensionalen Unterkieferbewegung und der Kieferschlusskraft. Bei diesem letzteren Einsatzgebiet wirkt sich besonders vorteilhaft aus, dass die Hygiene bleibend gesichert werden kann, sodass im sensiblen Mundinnenraum derartige "Geräte" sicher einsetzbar sind. Dabei ist sowohl der Patient geschützt wie auch die Vorrichtung als solche, sodass ein vorteilhafter Doppeleffekt zu verzeichnen ist. Die Dehnungsmessstreifen werden nicht auf dem Grundkörper geknickt, sondern vorteilhaft nur gebogen, sodass sie ihre Funktionsfähigkeit voll behalten. Das Elastizitätsmodul ist vorteilhaft auf die Dehnungsmessstreifen einstellbar.

Weitere Einzelheiten und Vorteile des Erfindungsgegenstandes ergeben sich aus der nachfolgenden Beschreibung der zugehörigen Zeichnung, in der ein bevorzugtes Ausführungsbeispiel mit den dazu notwendigen Einzelheiten und Einzelteilen dargestellt ist. Es zeigen:
- Figur 1: einen Schnitt durch ein Gehäuse mit Sensor,
- Figur 2: eine Draufsicht auf ein Gehäuse mit freigelegtem Grundkörper,
- Figur 3: eine Einzelheit in Form eines Druckvermittlers,
- Figur 4: einen weiteren Schnitt durch das Gehäuse mit Sensoren mit unmittelbar auf den Grundkörper aufgelegter Ausgleichsplatte,
- Figur 5: die Unteransicht einer Ausgleichsplatte und
- Figur 6: ein Gebiss mit dem Oberkiefer zugeordneten Bewegungs- und/oder Kraftinitiator und dem Unterkiefer zugeordneter Messeinrichtung.

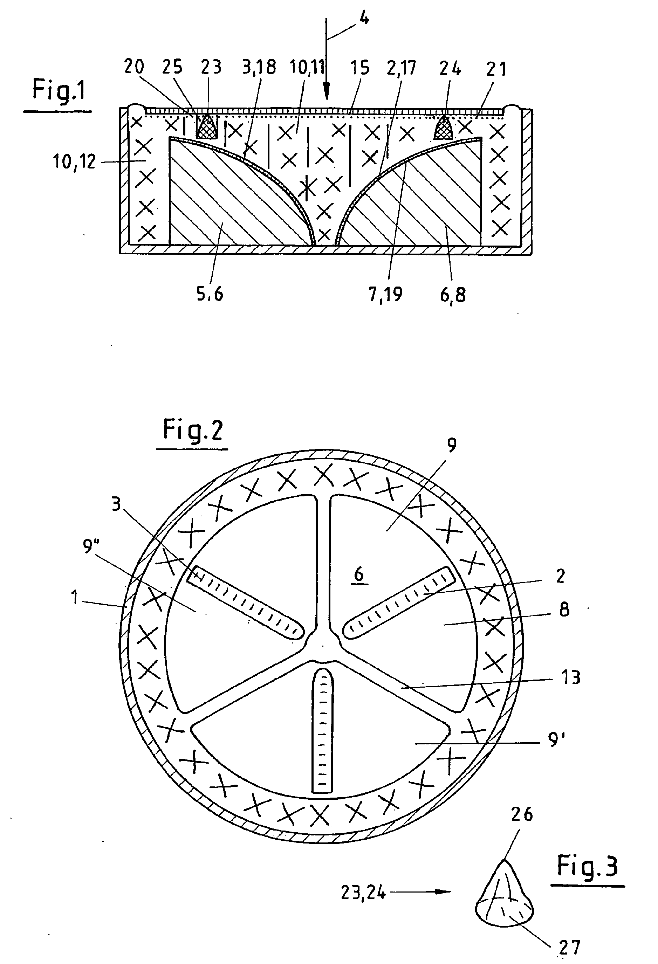

Figur 1 zeigt ein Gehäuse 1 mit innen liegenden Sensoren 2, 3, die über einen auf eine Ausgleichsplatte 15 einwirkenden Bewegungs- und/oder Kraftinitiator 4 beeinflussbar sind. Dazu sind die Sensoren 2, 3 einem Grundkörper 5 zugeordnet, der aus einem elastischem Kunststoff 6 besteht. Dieser Grundkörper 5 ist besonders geformt, wobei durch eine spezielle Kurvengeometrie in Kombination mit einer bestimmten Shore-Härte die Funktion von Biegeelementen 8 erreicht wird. Der Grundkörper 5 ist dazu mit einer Kurvenfläche 7 dieser bestimmten Kurvengeometrie ausgerüstet, wobei auf dieser Kurvenfläche 7 die Sensoren 2, 3 angeordnet sind und zwar dieser Kurvenfläche 7 angepasst.

Der Grundkörper 5 ist dabei so ausgebildet, dass er mehrere Kammern 9, 9',9" bildet, also eigentlich Teilkörper, die durch Spalte 13 beabstandet sind, wobei mittig dieser Kammern 9, 9', 9" die schon mehrfach erwähnten Sensoren 2, 3 angeordnet sind. Entsprechendes verdeutlicht Figur 2, wobei auch deutlich wird, dass dieser Grundkörper 5 mit seinen drei Kammern 9 oder auch mehr von einem Dichtkunststoffkörper 10 eingefasst ist. Besser ist noch Figur 1 zu entnehmen, dass dieser Dichtkunststoffkörper 10 aus einem Ringkörper 12 besteht sowie einem von oben aufgestülpten Kegelstück 11, das dabei dafür sorgt, dass die Sensoren 2, 3 in ihrer vorgegebenen Position gesichert angeordnet werden und auch angeordnet bleiben.

Bei den Sensoren 2, 3 handelt es sich im dargestellten Ausführungsbeispiel um Dehnungsmessstreifen 17, 18, die am oberen oder auch am unteren Ende Ansatzpunkte für Kabel aufweisen, um so die ermittelten Werte sicher abgreifen und dann der Auswerteeinheit, die hier nicht dargestellt ist, zuleiten zu können. Wichtig ist dabei, dass sowohl die hier nicht dargestellten Anschlussstellen wie auch der bogenförmige Teil 19 durch das Kegelstück 11 abgedeckt ist, sodass die von dem Kegelstück 11 von der Ausgleichsplatte 15 aufgenommenen Kräfte sicher übertragen werden können und zwar auf die entsprechend lang und besonders angeordneten Dehnungsmessstreifen 17, 18.

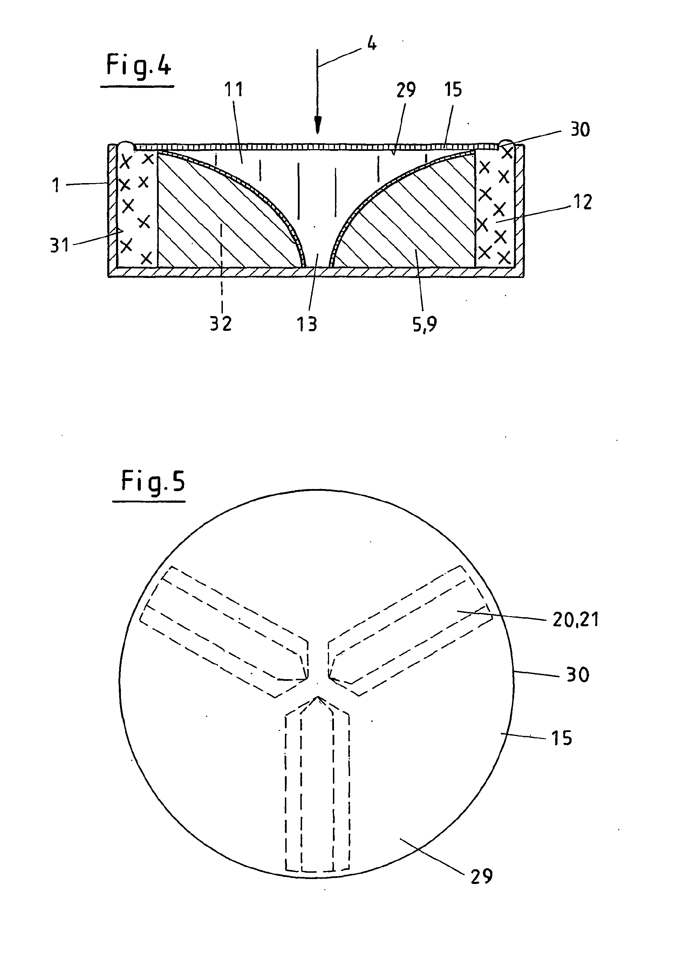

In der Region 20 oberhalb der Sensoren 2, 3 auf dem bogenförmigen Teil 19 ist ein Silikonöl-Schicht 21 aufgebracht. Entsprechendes ist in Figur 1 angedeutet. Ergänzend dazu zeigt Figur 5 eine Möglichkeit, wie diese Silikonöl-Schicht 21 auch nur in einzelnen Sektoren auf der Unterseite 29 der Ausgleichsplatte 15, die als verwindungsfreie Membran ausgebildet ist, verteilt werden kann.

Figur 1 zeigt weiter die Anordnung von Druckvermittlern 23, 24, deren vergrößerte Wiedergabe auch in Figur 3 gezeigt ist. Diese Druckvermittler 23, 24 sind in das Kegelstück 11 eingebettet, vorzugsweise in eine entsprechende Ausnehmung 25, wobei auf der Spitze 26 der Druckvermittler 23, 24 die Ausgleichsplatte 15 aufliegt. Eine gezielte Weiterleitung der auf die Ausgleichsplatte einwirkenden Kräfte und damit auch die Bewegungen können so sicher übertragen werden und zwar über die Sensoren 2, 3 bzw. die Dehnungsmessstreifen 17, 18 auf die hier nicht dargestellte Auswerteeinheit.

Figur 1 verdeutlicht die besondere Ausbildung der Druckvermittler 23, 24, die über eine entsprechende Spitze 26 verfügen, während die Grundfläche als großflächige Grundfläche 27 ausgeführt ist. Eine genaue Übertragung der Kräfte auch bleibend sicher ist somit gegeben. Auf der rechten Seite der Figur 1 ist angedeutet, dass auch andere Möglichkeiten der Anordnung der Druckvermittler 23, 24 gegeben sind, nur dass die Ausgleichsplatte 15 auch bei dieser Ausführung zweckmäßigerweise auf den drei Punkten, also den Spitzen 26 der drei Druckvermittler 23, 24 aufliegt.

Anders als nach der Darstellung von Figur 1 zeigt Figur 4 eine Ausbildung, bei der auf die Druckvermittler 23 und eine entsprechende Zwischenschicht des Kegelstückes 11 verzichtet ist. Hier liegt die Ausgleichsplatte 15 praktisch auf den Endbereichen des aus elastischem Kunststoff 6 bestehenden Grundkörpers 5 auf. Vor allem aber stützt sich die Ausgleichsplatte 15 auf dem ebenfalls elastisch ausgebildeten Ringkörper 12 ab, wobei durch die besondere Ausbildung sichergestellt ist, dass die ermittelten Bewegungen und Kräfte sicher auf die Sensoren 2, 3 übertragen werden. Figur 1 und auch Figur 4 verdeutlichen, dass der gesamte Innenraum 32 des Gehäuses 1 durch die verschiedenen Kunststoff- und Messteile ausgefüllt ist. Bei der aus Figur 4 ersichtlichen Ausführung sind die Unterseite 29 der Ausgleichsplatte 15 sowie deren Rand 30 und auch die Innenwand 31 des Gehäuses 1 mit dem Ringkörper 12 verschweißt oder verklebt, um die gewünschte absolute Dichtigkeit in diesem Bereich auch auf Dauer sicherzustellen.

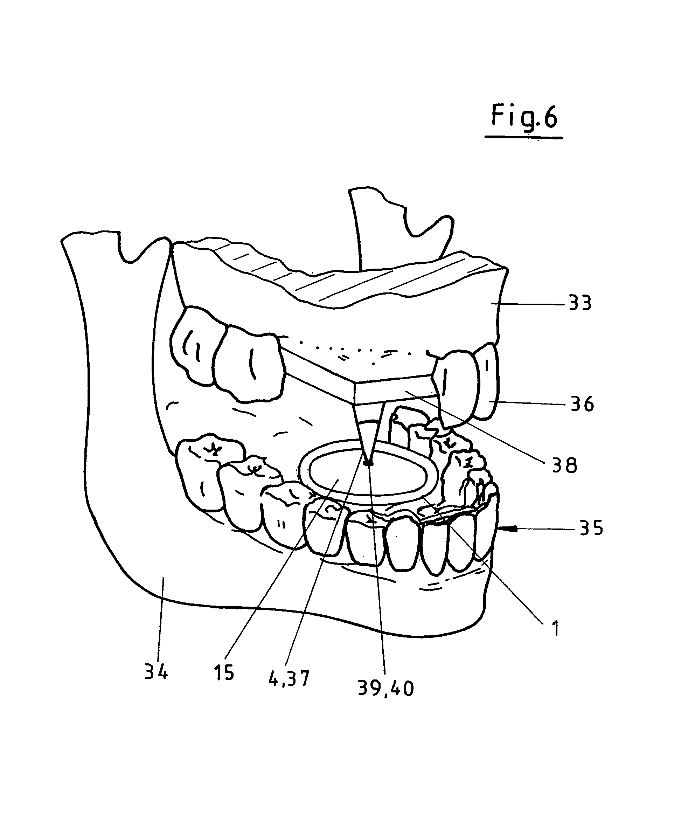

Figur 6 zeigt eine Verwendung im Bereich der Zahnmedizin, wobei hier der Bewegungs- und/oder Kraftinitiator 4 in Form eines Druckstiftes 37 einer Halteplatte 38 im Oberkiefer 33 zugeordnet ist. Die eigentliche Vorrichtung, d. h. das Gehäuse 1 ist dem Unterkiefer 34 zugeordnet, wobei das Gebiss 35 mit den Zähnen 36 nur teilweise wiedergegeben wird.

Die Spitze des Druckstiftes 37 bzw. des Bewegungs- und/oder Kraftinitiators 4 ist mit 39 bezeichnet und weist in aller Regel ein Kugellager 40, um so eine gleichmäßige Belastung und Auflastung auf der Ausgleichsplatte 15 zu erreichen. Mit dem Hin- und Hergehen dieses Druckstiftes 37 auf der Ausgleichsplatte 15 erreicht man eine entsprechende Beeinflussung des Kegelstücks 11 und des Grundkörpers 5, die hier nicht dargestellt sind, die aber die Weiterleitung der entsprechenden Kräfte auf die Sensoren 2, 3 möglich machen. Bezüglich weiterer Einzelheiten wird auf die weiter vom wiedergegebenen Ausführungen hingewiesen, wobei dieser hier wiedergegebene Einsatzfall schon andeutet, dass eine entsprechende Vorrichtung auch in ähnlich gelagerten Einsatzbereichen wirksam zum Einsatz kommen kann.

## Patentansprüche

1. Vorrichtung zur Ermittlung von Kraft und/oder Weg sich bewegender Gegenstände, Körper oder Körperteile bestehend aus einem Gehäuse (1) mit eingelagerten, von einer Ausgleichsplatte (15) beeinflussbaren Sensoren (2, 3) und einem auf die Ausgleichsplatte (15) einwirkenden, dem Gegenstand, Körper oder Körperteil zugeordneten Bewegungs- und/oder Kraftinitiator (4)
**dadurch gekennzeichnet,**
**dass** die Sensoren (2, 3) einem mehrfach gekammerten Grundkörper (5) aus elastischem Kunststoff (6) zugeordnet sind, der durch eine spezielle Kurvengeometrie in Kombination mit einer bestimmten Shore-Härte die Funktion von Biegeelementen (8) aufweisend ausgeführt ist, die die Sensoren (2, 3) aufnehmen und die zusammen mit den Sensoren (2, 3) von einem zweiten, das Gehäuse (1) ergänzend ausfüllenden, elastischen Dichtkunststoffkörper (10) eingefasst sind.

2. Vorrichtung nach Anspruch 1,
**dadurch gekennzeichnet,**
**dass** der Grundkörper (5) mehrfach, zumindest dreifach gekammert ist.

3. Vorrichtung nach einem der vorhergehenden Ansprüche,
**dadurch gekennzeichnet,**
**dass** das Gehäuse (1) dosenförmig ausgebildet und eine feste Hülle darstellend aus Metall oder einem Hartkunststoff gefertigt ist.

4. Vorrichtung nach einem der vorhergehenden Ansprüche,
**dadurch gekennzeichnet,**
**dass** die Sensoren (2, 3) als Dehnungsmessstreifen (17,18) ausgebildet und auf den bogenförmigen Teil (19) des Grundkörpers (5) aufgebracht sind.

5. Vorrichtung nach Anspruch 4,
**dadurch gekennzeichnet,**
**dass** die Dehnungsmessstreifen (17, 18) auf den bogenförmigen Teil (19) des Grundkörpers (5) aufgelegt, diesem angeformt und dann über ein Kegelstück (11) des Dichtkunststoffkörpers (10) darauf fixiert sind.

6. Vorrichtung nach einem der vorhergehenden Ansprüche,
**dadurch gekennzeichnet,**
**dass** die Dehnungsmessstreifen (17, 18) mittig der Kammern (9) auf den Grundkörper (5) aufgebracht sind.

7. Vorrichtung nach einem der vorhergehenden Ansprüche,
**dadurch gekennzeichnet,**
**dass** der Dichtkunststoffkörper (10) aus einem mittleren, in den Grundkörper (5) eingepassten Kegelstück (11) und einem den Grundkörper (5) einfassenden Ringkörper (12) besteht.

8. Vorrichtung nach Anspruch 7,
**dadurch gekennzeichnet,**
**dass** der Dichtkunststoffkörper (10) oder dessen Ringkörper (12) zugleich die Ausgleichsplatte (15) fixierend eingebracht, vorzugsweise sie einklebend ausgebildet ist.

9. Vorrichtung nach einem der vorhergehenden Ansprüche,
**dadurch gekennzeichnet,**
**dass** der Grundkörper (5) und der Dichtkunststoffkörper (10) aus kalthärtendem, additionsvernetzendem Silikon entsprechend abgestimmter Zusammensetzung hergestellt sind.

10. Vorrichtung nach Anspruch 7,
**dadurch gekennzeichnet,**
**dass** der die Ausgleichsplatte (15) fixierende Ringkörper (12) ein dieser und dem Gesamtsystem die notwendige Biegefreiheit belassendes Elastizitätsmodul aufweist.

11. Vorrichtung nach einem der vorhergehenden Ansprüche,
**dadurch gekennzeichnet,**
**dass** die Ausgleichsplatte (15) als verwindungsfreie Membran aus Metall, Keramik und/oder Kunststoff bestehend ausgebildet ist.

12. Vorrichtung nach Anspruch 7,
**dadurch gekennzeichnet,**
**dass** die Region (20) oberhalb der vom Kegelstück (11) eingefassten Sensoren (2, 3) eine hohe Gleitmöglichkeit aufweisend ausgebildet ist.

13. Vorrichtung nach Anspruch 12,
**dadurch gekennzeichnet,**
**dass** in den Regionen (20) zwischen Ausgleichsplatte (15) und Kegelstück (11) eine Silikonöl-Schicht (21) aufgebracht ist.

14. Vorrichtung nach einem der vorhergehenden Ansprüche,
**dadurch gekennzeichnet,**
**dass** oberhalb der Sensoren (2, 3) im Kegelstück (11) kugelförmige oder kegelförmige Druckvermittler (23, 24) angeordnet sind.

15. Vorrichtung nach Anspruch 14,
**dadurch gekennzeichnet,**
**dass** die Druckvermittler (23, 24) in Ausnehmungen (25) im Kegelstück (11) angeordnet sind.

16. Vorrichtung nach Anspruch 7,
**dadurch gekennzeichnet,**
**dass** der Ringkörper (12) des Dichtkunststoffkörpers (10) mit der Unterseite (29) und dem Rand (30) der Ausgleichsplatte (15) und mit der Innenwand (31) des Gehäuses (1) den Innenraum (32) des Gehäuses (1) voll abdichtend verklebt ist.

## Claims

1. A device for determining the force and/or path of moving objects, bodies or body parts, consisting of a housing (1) with incorporated sensors (2, 3), which can be influenced by a compensation plate (15), and a motion and/or force initiator (4) acting on the compensation plate (15) and associated with the object, body or body part,
**characterized in that**
the sensors (2, 3) are associated with a basic body (5) of elastic plastics material (6) which is chambered several times and is constructed by means of a special curve geometry in combination with a specific Shore hardness so as to have the function of bending elements (8) which accommodate the sensors (2, 3) and which, together with the sensors (2, 3), are encompassed by a second elastic sealing plastics body (10) which additionally fills the housing (1).

2. The device according to Claim 1,
**characterized in that**
the basic body (5) is chambered several times, at least three times.

3. The device according to any one of the preceding Claims,
**characterized in that**
the housing (1) is can-shaped and, representing a rigid shell, is made of metal or a hard plastics material.

4. The device according to any one of the preceding Claims,
**characterized in that**
the sensors (2, 3) are formed as strain gauges (17, 18) and applied to the arcuate part (19) of the basic body (5).

5. The device according to Claim 4,
**characterized in that**
the strain gauges (17, 18) are laid on the arcuate part (19) of the basic body (5), moulded onto this and then fixed thereto by way of a conical piece (11) of the sealing plastics body (10).

6. The device according to any one of the preceding Claims,
**characterized in that**
the strain gauges (17, 18) are applied to the basic body (5) in the centre of the chambers (9).

7. The device according to any one of the preceding Claims,
**characterized in that**
the sealing plastics body (10) consists of a central conical piece (11), which fits into the basic body (5), and an annular body (12), which encompasses the basic body (5).

8. The device according to Claim 7,
**characterized in that**
the sealing plastics body (10) or the annular body (12) thereof is introduced so as to fix the compensation plate (15), preferably stick it in, at the same time.

9. The device according to any one of the preceding Claims,
**characterized in that**
the basic body (5) and the sealing plastics body (10) are made of cold-curing, addition-crosslinking silicone of an appropriately adapted composition.

10. The device according to Claim 7,
**characterized in that**
the annular body (12) fixing the compensation plate (15) has a modulus of elasticity which leaves the latter and the overall system the necessary bending freedom.

11. The device according to any one of the preceding Claims,
**characterized in that**
the compensation plate (15) is formed as a torsion-free membrane of metal, ceramic material and/or plastics material.

12. The device according to Claim 7,
**characterized in that**
the region (20) above the sensors (2, 3) encompassed by the conical piece
(11) is formed so as to have a high sliding facility.

13. The device according to Claim 12,
**characterized in that**
a silicone oil layer (21) is applied in the regions (20) between the compensation plate (15) and the conical piece (11).

14. The device according to any one of the preceding Claims,
**characterized in that**
spherical or conical pressure mediators (23, 24) are disposed above the sensors (2, 3) in the conical piece (11).

15. The device according to Claim 14,
**characterized in that**
the pressure mediators (23, 24) are disposed in recesses (25) in the conical piece (11).

16. The device according to Claim 7,
**characterized in that**
the annular body (12) of the sealing plastics body (10) is stuck to the underside (29) and the edge (30) of the compensation plate (15) and to the inner wall (31) of the housing (1) so as to completely seal the interior space (32) of the housing (1).

## Revendications

1. Dispositif pour la détermination de la force et/ou du trajet d'objets, de corps ou de parties de corps en mouvement, se composant d'un boîtier (1) avec des capteurs (2, 3) intercalés, influençables par une plaque de compensation, (15) et un initiateur de mouvement et/ou de force (4) affecté à l'objet, au corps ou à la partie de corps et agissant sur la plaque de compensation (15).
**caractérisé en ce que**
les capteurs (2, 3) sont affectés à un corps de base (5) à plusieurs chambres en matière plastique élastique (6) qui est réalisé de manière à présenter, par une géométrie de courbe spéciale en combinaison avec une dureté Shore précise, la fonction d'éléments flexionnels (8) qui accueillent les capteurs (2, 3) et qui sont entourés ensemble avec les capteurs (2, 3) par un deuxième corps en matière plastique étanche (10) élastique de remplissage complétant le boîtier (1).

2. Dispositif selon la revendication 1,
**caractérisé en ce que**
le corps de base (5) comporte plusieurs, au moins trois chambres.

3. Dispositif selon l'une quelconque des revendications précédentes,
**caractérisé en ce que**
le boîtier (1) est réalisé en forme de boîte et est fabriqué en métal ou en une matière plastique dure représentant une enveloppe solide.

4. Dispositif selon l'une quelconque des revendications précédentes,
**caractérisé en ce que**
les capteurs (2, 3) sont formés comme jauges extensométriques (17, 18) et sont rapportés sur la partie arquée (19) du corps de base (5).

5. Dispositif selon la revendication 4,
**caractérisé en ce que**
les jauges extensométriques (17, 18) sont posées sur la partie arquée (19) du corps de base (5), sont adaptées à la forme de ce dernier, puis y sont fixées par l'intermédiaire d'une pièce conique (11) du corps en matière plastique étanche (10).

6. Dispositif selon l'une quelconque des revendications précédentes,
**caractérisé en ce que**
les jauges extensométriques (17, 18) sont rapportées au centre des chambres (9) sur le corps de base (5).

7. Dispositif selon l'une quelconque des revendications précédentes,
**caractérisé en ce que**
le corps en matière plastique étanche (10) se compose d'une pièce conique (11) centrale adaptée dans le corps de base (5) et d'un corps annulaire (12) qui entoure le corps de base (5).

8. Dispositif selon la revendication 7,
**caractérisé en ce que**
le corps en matière plastique étanche (10) ou son corps annulaire (12) est encastré de manière à fixer en même temps la plaque de compensation (15), de préférence en la collant.

9. Dispositif selon l'une quelconque des revendications précédentes,
**caractérisé en ce que**
le corps de base (5) et le corps en matière plastique étanche (10) sont fabriqués en silicone de composition accordée en conséquence durcissant à froid et réticulant par addition.

10. Dispositif selon la revendication 7,
**caractérisé en ce que**
le corps annulaire (12) fixant la plaque de compensation (15) présente un module d'élasticité qui laisse à cette dernière et au système entier la liberté de flexion nécessaire.

11. Dispositif selon l'une quelconque des revendications précédentes,
**caractérisé en ce que**
la plaque de compensation (15) est formée comme membrane anti-torsion en métal, céramique et/ou matière plastique.

12. Dispositif selon la revendication 7,
**caractérisé en ce que**
la région (20) en amont des capteurs (2, 3) entourés par la pièce conique (11) présente une grande possibilité de glissement.

13. Dispositif selon la revendication 12,
**caractérisé en ce que**
une couche de silicone (21) est appliquée dans les régions (20) entre la plaque de compensation (15) et la pièce conique (11).

14. Dispositif selon l'une quelconque des revendications précédentes,
**caractérisé en ce que**
sont disposés en amont des capteurs (2, 3), dans la pièce conique (11), des médiateurs de pression (23, 24) sphériques ou coniques.

15. Dispositif selon la revendication 14,
**caractérisé en ce que**
les médiateurs de pression (23, 24) sont disposés dans des évidements (25) dans la pièce conique (11).

16. Dispositif selon la revendication 7,
**caractérisé en ce que**
le corps annulaire (12) du corps en matière plastique étanche (10) est collé à la face inférieure (29) et au bord (30) de la plaque de compensation (15) et à la paroi intérieure (31) du boîtier (1) de manière à étanchéifier complètement l'espace intérieur (32) du boîtier (1).
